# EUROPEAN PATENT SPECIFICATION

(11) **EP 0 562 316 B1**
(45) Date of publication and mention of the grant of the patent: **13.12.1995**
(21) Application number: 93103411.0
(22) Date of filing: 03.03.1993
(51) Int. Cl.: A61F 7/03

(54) **A disposable foot warmer**
Fusswärmer zum einmaligen Gebrauch
Chauffe-pieds jetable

(30) Priority: 27.03.1992 JP 25917/92 U
(43) Date of publication of application: 29.09.1993
(73) Proprietor: Kiyohara, Takashi, Nishinomiya-shi, Hyogo-ken (JP)
(72) Inventor: Kiyohara, Takashi, Nishinomiya-shi, Hyogo-ken (JP)
(74) Representative: Menges, Rolf, Dipl.-Ing.

(56) References cited:
- EP-A- 0 045 642
- US-A- 1 586 546
- US-A- 2 573 791
- US-A- 4 249 319

## Description

This invention relates to a disposable foot warmer which is capable of warming a foot when being applied to socks or stockings.

Previously, disposable foot warmers comprised a flexible thin inner bag made of materials having gas permeability and an outer bag made of materials having no gas permeability in which the inner bag was vacuum packaged. The inner bag contained a mixture of powders, main components of which were ferrous powders capable of generating heat when being oxidized with atmospheric oxygen and powders of pro-oxidants for the ferrous powders. Besides, the inner bag had a front side made of materials having gas permeability and a back side made of materials to which a layer of an adhesive was applied, and the surface of the layer of adhesive was releasably covered by a releasing paper. The inner bag to be taken out of the outer bag was a thin bag formed almost in the shape of a rectangle having the width of about 5.5 centimeters and the length of about 8.5 centimeters, and the largest thickness of the inner bag was about 0.7 centimeters. The inner bag was made so that it may warm a foot, when being applied to socks or stockings at their portion confronting so-called ball of the sole of a foot. However, the disposable foot warmers had drawbacks mentioned below.

Since the inner bag came in contact with the sole of a foot at its portion which was affected pressure, when a user who applied the inner bag to his socks or sto ckings put on shoes and walked, the user felt tight and uncomfortable, and he felt as if there was something in his shoes, and he was injured in the sole of his foot. Accordingly, it was difficult for the user to walk for a long time or to take hard exercise and work. Besides, the inner bag easily slipped off from the position at which it had been applied to, and the inner bag was easily removed from socks or stockings.

US-A-4249 319 relates to "a heat insulating insert for footwear", in which one of paper bags containing exothermic agent is inserted into a porous bag disposed in a portion of the base sheet corresponding to the plantar arch of a foot to warm the foot. The exothermic agent comprises a mixture of metal powder such as iron powder and oxidizing catalyst such as active carbon, saline matter or moisture, and the paper bag is taken out of an airtight bag such as an aluminum foil bag. The paper bag does not have a layer of an adhesive and a releasing paper releasably affixed to the layer. And accordingly, the paper bag cannot be affixed to socks or stockings.

US-A-1 586 546 relates to "a therapeutic thermophore" which is used as being inserted into the space of the plantar arch of a foot. The thermophore includes an out-lining shell of thin metal, enclosing a heat receiving, storing and dispensing substance. Accordingly, Wyeth does not relates to a foot warmer containing ferrous powders and an oxidizing catalyst.

US-A-2 578 791 discloses "a heat applying bandage" which comprises a layer of flexible material having one adhesive face on which there is secured an enclosure containing an exothermic mixture. However, the heat applying bandage is not intended for being applied to socks or stockings at their portion confronting the plantar arch of a foot.

EP-A-0 045 642 Mitsubishi relates to "an exothermic body for use of an insole of shoes and so forth with elasticity, softness and flexibility".

An object of this invention is to provide a disposable foot warmer comprising an inner bag which does not make a user feel tight and uncomfortable, which does not make a user feel as if there were something in his shoes, which does not hurt the sole of his foot, and which enables a user to walk for a long time and to take hard exercise or work, when the user puts on shoes and walks, after the inner bag has been applied to his socks or stockings.

To accomplish the foregoing object, this invention provides a disposable foot warmer as defined in claim 1

In a preferred embodiment, the inner bag is shaped like a half-moon or ellipse having an area which does not protrude out of the space of the plantar arch of the foot.

The invention will be clarified with reference to the accompanying drawings.
Fig. 1 is a planar view of an embodiment of a disposable foot warmer according to the present invention;
Fig. 2 is an enlarged sectional view showing the embodiment on the line A - A of Fig. 1, as the middle portion is omitted;
Fig. 3 is a diagram of a side view showing a working condition of the inner bag of the embodiment of Fig. 1, which is applied to socks at their portion confronting the plantar arch, as shoes are put on;
Fig. 4 is a diagram of a planar view showing a working condition of the inner bags of the embodiment of Fig. 1, which are applied to socks at their portion confronting the plantar arch and at their portion confronting a depressed portion between the tip of toes and the instep, as shoes are put on;
Fig. 5 is a planar view of another embodiment of a disposable foot warmer according to the present invention;
Fig. 6 is an enlarged sectional view showing the embodiment on the line B - B of Fig. 5, as the middle portion is omitted; and
Fig. 7 is a diagram of a planar view showing a working condition of the inner bags of the embodiment of Fig. 5, which are applied to socks at their portion confronting the plantar arch and at their portion confronting a depressed portion between the tip of toes and the instep, as shoes are put on.

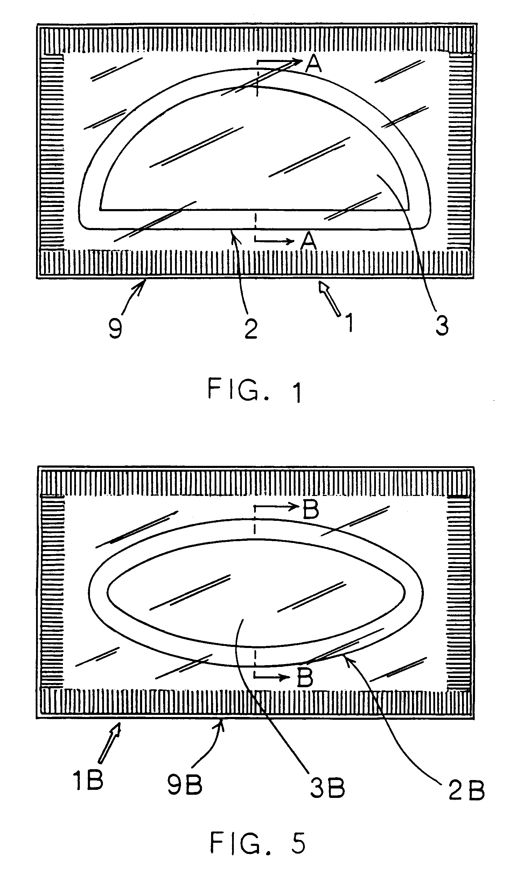

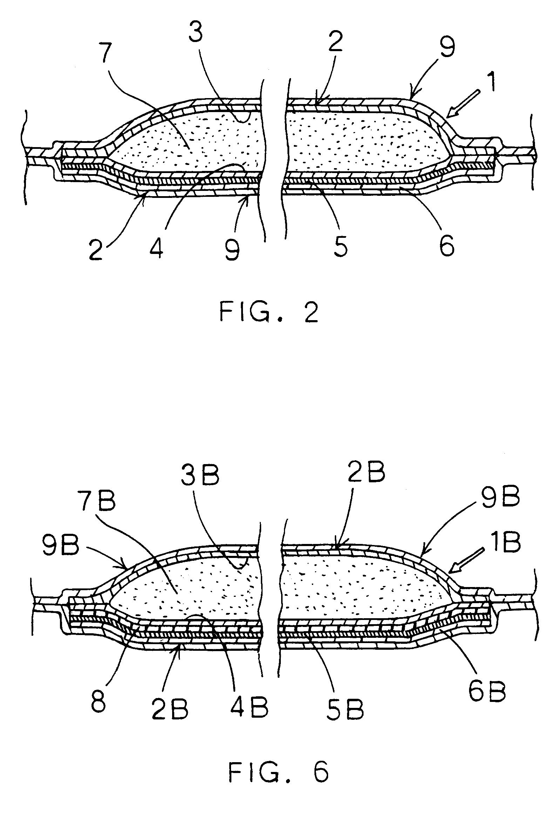

A preferred embodiment according to the invention is shown in Figs. 1 to 4.

A disposable foot warmer 1 according to the embodiment comprises an inner bag 2 which contains a mixture of powders 7 comprising iron filings, moisture, salt, powders of activated carbon and wood flour and an outer bag 9 in which the inner bag 2 has been vacuum packaged.

The inner bag 2 comprises a front side made of a flexible non-woven fabric 3 having gas permeability and a back side made of a flexible non-woven fabric 4 to which a layer of an adhesive 5 has been applied, and which has no gas permeability. A releasing paper 6 is releasably affixed to the surface of the layer of the adhesive 5 applied to the back side of the flexible non-woven fabric 4.

The inner bag 2 is shaped like a half-moon which has the largest width of about 4.5 centimeters and the largest length of about 8.0 centimeters, and the thickness of the inner bag 2 is about 0.82 to 1.2 centimeters. The inner bag 2 has an area and a thickness which do not protrude out of the space of the plantar arch 14 of an adult foot 13.

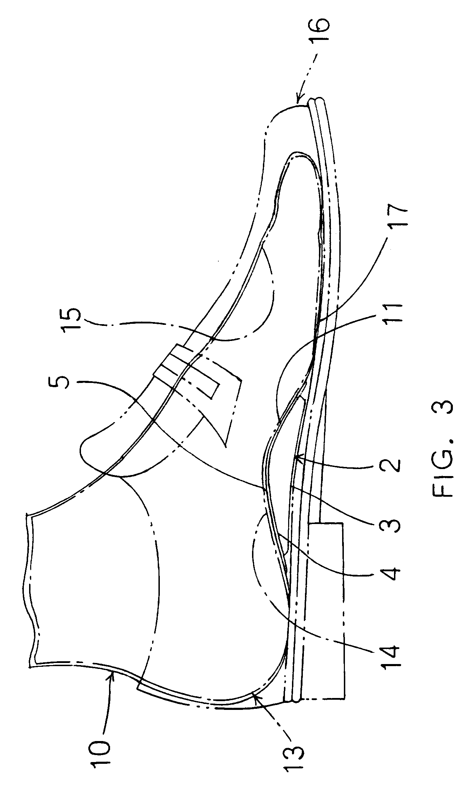

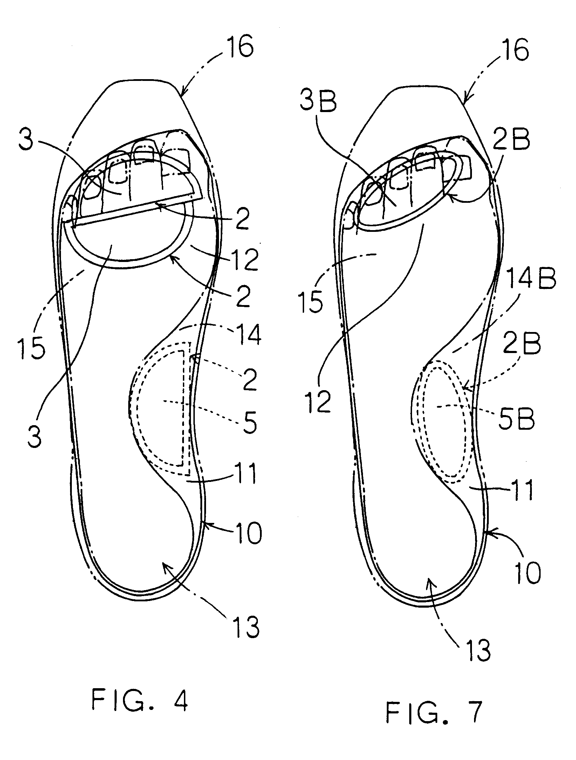

Because of the disposable foot warmer 1 according to this embodiment being constituted as above-mentioned, after the outer bag 9 is teared to be opened, the inner bag 2 can be taken out of the outer bag 9. After the releasable paper 6 has been removed, as shown in Figs. 3 and 4, the inner bag 2 is applied to socks 10 at their portion 11 confronting the plantar arch 14 of the foot 13, and then, shoes 16 are put on.

Besides, as shown in Fig. 4, the inner bag 2 may be applied to socks 10 at their portion 12 confronting a depressed portion 15 of a foot 13 between the tip of toes and the instep, and then, shoes 16 are put on.

The inner bag 2 applied to the portion 11 of socks 10 or the inner bag 2 applied to the portion 12 of socks 10 generates heat when the mixture of powders 7 are oxidized with air in the shoes 16. Thus, the inner bag 2 can warm the foot 13.

Another embodiment of the invention is shown in Figs. 5 - 7.

A disposable foot warmer 1B according to the embodiment comprises an inner bag 2B containing a mixture of powders 7B having the same composition as that mentioned in before-mentioned example and an outer bag 9B in which the inner bag 2B has been vacuum packaged.

The inner bag 2B has a front side made of a flexible non-woven fabric 3B having gas permeability and a back side which comprises a flexible non-woven fabric 4B having no gas permeability, a sheet of foamed polyethylene 8 adhered to the non-woven fabric 4B and a layer of an adhesive 5B applied to the sheet 8. A releasing paper 6B is releasably affixed to the surface of the layer of the adhesive 5B applied to the sheet of foamed polyethylene 8.

Since the sheet of foamed polyethylene 8 is adhered to the non-woven fabric 4B, it is possible for the temperature of the oxidation of the mixture of powders 7B to be sharply raised. In addition, the sheet 8 can prevent a user from getting burnt so called at low temperature, and the sheet 8 is useful for lessening undesirable feeling which the user feels when the mixture of powders 7B have formed a hard mass at the end of the reaction of the generation of heat.

The inner bag 2B is shaped like an elongated ellipse which has the length of about 8.5 centimeters and the width of about 3.5 centimeters, and the thickness of the inner bag 2B is about 0.51 to 0.91 centimeters. The inner bag 2B has an area and a thickness which do not protrude out of the space of the plantar arch 14 of an adult foot 13.

Since the disposable foot warmer 1B according to this embodiment is constituted as above-mentioned, the inner bag 2B can be taken out of the outer bag 9B in which the inner bag 2B has been vacuum packaged. After the releasing paper 6B has been removed, as shown in Fig. 7, the inner bag 2B can be applied to soaks 10 at their portion 11 confronting the plantar arch 14 of an adult foot 13, and then shoes 16 are put on.

Besides, the inner bag 2B may be applied to socks 10 at their portion 12 confronting the depressed portion 15 of a foot 13 between the tip of toes and the instep, and then, shoes 16 are put on. The inner bag 2B applied to socks 10 at their portion 11 or the inner bags 2B applied to socks at their portions 11 and 12 generate heat when being oxidized with air in the shoes 16. Thus, the inner bag 2B can warm the foot 13.

According to this invention, the inner bag 2 or 2B can be applied to socks or stockings 10 at their portion 11 confronting the plantar arch 14 of a foot 13, and if there is a surplus space in shoes, the inner bag 2 or 2B may be applied to socks or stockings 10 at their portion 12 confronting the depressed portion 15 of a foot 13 between the tip of toes and the instep.

Since the inner bag 2 or 2B applied to socks or stockings 10 at their portion 11 confronting the plantar arch 14 enters the space between the plantar arch 14 and an insole of shoes 16, it is possible for the inner bag 2 or 2B to be made with a considerable thickness, and the inner bag 2 or 2B does not make a user feel tight of his shoes during walking. In addition, since the inner bag 2 or 2B enters the space between the plantar arch 14 of a foot 13 and the insole of shoes 16, and since the inner bag 2 or 2B is not applied to socks or stockings 10, for instance, at their portion 17 confronting so called ball of the sole of a foot 13 which is affected pressure, the inner bag does not make the user feel tight and uncomfortable, and the inner bag does not hurt the sole of a foot, and the inner bag does not make a user feel as if there were something in his shoes. Furthermore, the inner bag does not slip off from the position to which the inner bag has been applied, and the inner bag is not removed from socks or stockings. Thus, the inner bag can endure walks, works and exercises for a long time.

Furthermore, in case of well-fitted woman's shoes, the woman's shoes did not have surplus spaces which enabled prior disposable foot warmers to be applied to socks or stockings. However, the disposable foot warmers according to this invention can be used even when such well-fitted woman's shoes are put on, because the disposable foot warmer is intended to be applied to socks or stockings at their portion confronting the plantar arch of a foot so that the inner bag may enter the space between the plantar arch and the insole of shoes.

## Claims

1. A disposable foot warmer (1, 1B) comprising a flexible inner bag (2, 2B) which is made of materials having gas permeability and which contains a mixture of powders (7, 7B), main components of which are ferrous powders capable of generating heat when being oxidized with atmospheric oxygen and powders of pro-oxidants for the ferrous powders and an outer bag (9, 9B) which is made of materials having no gas permeability and in which said inner bag (2, 2B) has been vacuum packaged, said inner bag (2, 2B) being capable of warming a foot (13) as being applied to socks or stockings (10) when the inner bag (2, 2B) is generating heat, after the inner bag (2, 2B) has been taken out of the outer bag (9, 9B), and after a releasing paper (6, 6B) has been removed from the surface of a layer of an adhesive (5, 5B), said disposable foot warmer (1, 1B) characterized in that the inner bag (2, 2B) is shaped to have an area and a thickness which do not protrude out of the space between the plantar arch (14) of a foot (13) and an insole of shoes (16), so that the inner bag (2, 2B) may be used as being applied to socks or stockings (10) at their portion (11) confronting the plantar arch (14) of the foot (13), and that the inner bag (2, 2B) has a front side made of materials having gas permeability and a back side made of materials which do not have gas permeability and to which the layer of an adhesive (5, 5B) has been applied whereby to enable the inner bag (2, 2B) to be applied to socks or stockings (10), so that heat emitted through the front side of the inner bag (2, 2B) may warm air in the shoes (16) and the insole of the shoes (16) whereby to warm the whole foot, and so that heat conducted to the plantar arch (14) through the back side of the inner bag (2, 2B) may warm the sole of the foot (13).

2. A disposable foot warmer (1) as claimed in claim 1, wherein the inner bag (2) is shaped like a half-moon.

3. A disposable foot warmer (1B) as claimed in claim 1, wherein the inner bag (2B) is shaped elliptically.

## Patentansprüche

1. Einwegfußwärmer (1, 1B) mit einem flexiblen inneren Beutel (2, 2B) der aus Materialien mit Gaspermeabilität hergestellt ist und eine Mischung von Pulvern (7, 7B) enthält, deren Hauptkomponenten eisenhaltige Pulver, welche zu Wärmeerzeugung fähig sind, wenn sie mit atmosphärischem Sauerstoff oxidiert werden, und Pulver von Pro-Oxidantien für die eisenhaltigen Pulver sind, und mit einem äußeren Beutel (9, 9B), der aus Materialien ohne Gaspermeabilität hergestellt ist und in den der innere Beutel (2, 2B) vakuumverpackt worden ist, wobei der innere Beutel (2, 2B) an Socken oder Strümpfen (10) angebracht zum Wärmen eines Fußes (13) fähig ist, wenn der innere Beutel (2, 2B) Wärme erzeugt, nachdem der innere Beutel (2, 2B) aus dem äußeren Beutel (9, 9B) herausgenommen worden ist und nachdem ein Abziehpapier (9, 9B) von der Oberfläche einer Klebstoffschicht (5, 5B) entfernt worden ist, wobei der Einwegfußwärmer (1, 1B) dadurch gekennzeichnet ist, daß der innere Beutel (2, 2B) so geformt ist, daß er eine Grundfläche und eine Dicke hat, welche nicht aus dem Raum zwischen dem Sohlengewölbe (14) eines Fußes (13) und einer Einlage von Schuhen (16) nach außen hervorstehen, so daß der innere Beutel benutzt werden kann, um an Socken oder Strümpfen (10) an deren Teil (11), welcher dem Sohlengewölbe (14) des Fußes (13) gegenüberliegt, angebracht zu werden, und daß der innere Beutel (2, 2B) eine aus Materialien mit Gaspermeabilität gefertigte Vorderseite und eine Rückseite hat, die aus Materialien ohne Gaspermeabilität gefertigt ist und an der die Klebstoffschicht (5, 5B) angebracht worden ist, wodurch ermöglicht wird, den inneren Beutel (2, 2B) an Socken oder Strümpfen (10) anzubringen, so daß durch die Vorderseite des inneren Beutels (2, 2B) ausgetretene Wärme die Luft in den Schuhen (16) und die Einlage der Schuhe (16) wärmen kann, um hierdurch den gesamten Fuß zu wärmen, und so daß durch die Rückseite des inneren Beutels (2, 2B) zu dem Fußsohlengewölbe (14) geleitete Wärme die Sohle des Fußes (13) wärmen kann.

2. Einwegfußwärmer (1) nach Anspruch 1, wobei der innere Beutel (2) wie ein Halbmond geformt ist.

3. Einwegfußwärmer (1B) nach Anspruch 1, wobei der innere Beutel (2B) elliptisch geformt ist.

## Revendications

1. Chauffe-pieds jetable (1, 1B) comprenant un sachet interne flexible (2, 2B) composé de matériaux perméables aux gaz et contenant un mélange de poudres (7, 7B), dont les composants principaux sont des poudres ferreuses susceptibles de produire de la chaleur lorsqu'elles sont oxydées avec de l'oxygène atmosphérique et des poudres favorisant l'oxydation desdites poudres ferreuses et une enveloppe externe (9, 9B) composée de matériaux non perméables aux gaz et dans laquelle ledit sachet interne (2, 2B) a été emballé sous vide, ledit sachet interne (2, 2B) étant susceptible de chauffer un pied (13) en l'appliquant à des chaussettes ou à des bas (10) lorsque le sachet interne (2, 2B) produit de la chaleur, après que le sachet interne (2, 2B) a été sorti du sachet externe (9, 9B) et après qu'un papier couché de séparation (6, 6B) a été enlevé de la surface d'une couche d'adhésif (5, 5B), ledit chauffe-pieds jetable (1, 1B) étant caractérisé en ce que le sachet interne (2, 2B) est formé de manière à avoir une surface et une épaisseur qui ne saillent pas de l'espace compris entre la voûte plantaire (14) d'un pied (13) et une semelle de chaussure (16), de sorte que le sachet interne (2, 2B) puisse être utilisé en l'appliquant aux chaussettes ou aux bas (10) a niveau de leur partie (11) en contact avec la voûte plantaire (14) du pied (13), et en ce que le sachet interne (2, 2B) présente une face avant composée de matériaux perméables aux gaz et une face arrière composée de matériaux non perméables aux gaz et auxquels la couche d'adhésif (5, 5B) a été appliquée, de manière à permettre l'application du sachet interne (2, 2B) aux chaussettes ou aux bas (10), de sorte que la chaleur transmise à travers la face avant du sachet interne (2, 2B) puisse chauffer l'air dans les chaussures (16) et la semelle des chaussures (16) de manière à chauffer tout le pied, et de sorte que la chaleur conduite vers la voûte plantaire (14) à travers la face arrière du sachet interne (2, 2B) puisse chauffer la plante du pied (13).

2. Chauffe-pieds jetable (1) selon la revendication 1, dans lequel le sachet interne (2) a la forme d'une demi-lune.

3. Chauffe-pieds jetable (1B) selon la revendication 1, dans lequel le sachet interne (2B) est de forme elliptique.
